# EUROPÄISCHE PATENTANMELDUNG

(11) **EP 0 916 368 A2**
(43) Veröffentlichungstag der Anmeldung: **19.05.1999**
(21) Anmeldenummer: 98119068.9
(22) Anmeldetag: 08.10.1998
(51) Int. Cl.: A61N 2/02

(54) **Magnetfeldtherapiegerät**

(30) Priorität: 18.11.1997 DE 19750962
(71) Anmelder: Schmidt, Kai-Oliver, 87437 Kempten (DE); Skischally, Wolfgang, 87700 Kempten (DE)
(72) Erfinder: Schmidt, Kai-Oliver, 87437 Kempten (DE); Skischally, Wolfgang, 87700 Kempten (DE)
(74) Vertreter: Hübner, Hans-Joachim, Dipl.-Ing.

(57) **Zusammenfassung**

Eine Magnetfeld-Therapiematte (12) weist in jeder ihrer beiden Längshälften eine schlangenförmige Litzenanordnung (18, 20) auf, die jeweils aus parallelen Strängen (22) und Verbindungsbögen (24) zusammengesetzt ist. Die parallelen Stränge (22) sind in einem engen Abstand von ca. 3 cm angeordnet, liegen exakt auf gleichem Niveau, etwa auf halber Mattendicke und erstrecken sich quer zur Längsrichtung der Matte. Die vier Enden der Litzenanordnungen (18, 20) sind zu einem gemeinsamen Mattenausgang (26) geführt und an ein mehradriges Kabel (14) angeschlossen, das zu einem Steuergerät (10) führt, das zwei getrennte Impulsgeber aufweist, deren Frequenzen unabhängig voneinander einstellbar sind. Bei jedem Impuls schreitet der Magnetfeldaufbau in jedem der beiden Mattenbereiche von oben nach unten fort. Dank des engen Abstandes der parallelen Litzenstränge (22) werden gleichmäßige Magnetfelder erzeugt.

## Beschreibung

Die Erfindung betrifft ein Magnetfeldtherapiegerät mit einer länglichen Matte, in der mehrere flexible, gleich breite, elektrisch leitende Litzenanordnungen je mit einer Vielzahl paralleler Stränge in Kanälen der Matte schlangenförmig verlegt und die parallelen Stränge der Litzenanordnungen rechtwinklig zur Längserstreckung der Matte orientiert und in Längsrichtung der Matte hintereinander angeordnet sind, deren Enden über Kabel an einen Stromimpulserzeuger angeschlossen oder anschließbar sind.

Ein derartiges Magnetfeldtherapiegerät zeigt die EP 0 239 098 A2. Die parallelen Stränge verlaufen in einer Litzenanordnung in Längsrichtung der Matte und in weiteren Litzenanordnungen längs und quer zur Matte in Form von Rechteck-Spiralen. Die Stränge sind in den Kanälen fixiert.

Die DE 40 04 682 A1 zeigt ein Magnetfeldtherapiegerät mit einer Decke, die eine Anzahl Litzenanordnungen aufweist, welche hier ausschließlich als spiralförmig gewickelte Induktionsspulen ausgebildet sind. Die Induktionsspulen bilden Gruppen, die in Mattenlängsrichtung hintereinander liegen. Die Stränge sind nicht parallel angeordnet und haben auch unterschiedliche Abstände.

Die CH 681 356 A zeigt eine weitere Ausführungsform einer Magnetfelddecke, bei der mehrere Magnetfelder nebeneinander und hintereinander angeordnet sind. Die Wicklungen sind hier dreieckförmig verlegt. Die Litzen der Induktionsspulen sind in der Decke festgelegt.

Die DE 41 08 437 A1 zeigt wiederum ein Magnetfeldtherapiegerät mit spiralförmigen Spulenanordnungen, die reihenweise in Gruppen zusammengeschaltet sind und zeitlich versetzt angesteuert werden. Die Erregung der Spulen erfolgt gegensinnig, also von beiden Enden der Matte zur Mattenmitte hin.

Die EP 0 392 626 schließlich zeigt ebenfalls ein Magnetfeldtherapiegerät, bei dem eine an einen Impulserzeuger angeschlossene Matte verwendet wird, die eine Anzahl Wickelspulen enthält, die einzeln oder in Gruppen aktivierbar sind, um Magnetfelder mit unterschiedlichen Intensitäten und Frequenzen zu erzeugen.

Aufgabe der Erfindung ist es, ein Therapiegerät der eingangs genannten Art so weiter auszubilden, daß der Therapieeffekt und die Haltbarkeit der Matte mit den empfindlichen, stromleitenden Strängen verbessert werden.

Diese Aufgabe wird bei einem Therapiegerät der eingangs genannten Art dadurch gelost, daß die parallelen Stränge der Litzenanordnungen rechtwinklig zur Längserstreckung der Matte orientiert und in allen Litzenanordnungen in exakt gleichem Abstand im Bereich von 2 bis 5 cm, insbesondere etwa 3 cm zueinander angeordnet und in den Kanälen längsbeweglich geführt sind, und daß die Verbindungsbogen der Litzenanordnungen auf einem vom Niveau der parallelen Stränge beabstandeten Niveau liegen.

Dank der parallelen Stränge quer zur Längsrichtung der Matte wird erreicht, daß bei jedem Stromimpuls der Magnetfeldaufbau von oben nach unten fortschreitet, was für die zu behandelnde Person einen erheblich höheren Therapieeffekt hat, als wenn der Magnetfeldaufbau von einer Seite der Matte zur anderen Seite hin oder radial zu vorgegebenen Zentren hin erfolgt. Es ist gefunden worden, daß der menschliche Körper für die Magnetfeldtherapiebehandlung im wesentlichen in drei Bereiche aufzuteilen ist, nämlich einen oberen Kopfbereich, einen mittleren Rumpfbereich und einen unteren Bereich für die Extremitäten. Die Frequenzen der Magnetfeldschwingungen sind in den einzelnen Bereichen unterschiedlich einzustellen, um optimale Therapieeffekte zu erzielen. So läßt sich vorzugsweise der Kopfbereich mit einer geringeren Frequenz beaufschlagen als der Therapiebereich für die Extremitäten. Für die Praxis ist eine Aufteilung der Matte in zwei Bereiche mit eigenen Litzenanordnungen ausreichend, obwohl es im Rahmen der Erfindung liegt, drei oder sogar mehr Litzenanordnungen in Längsrichtung der Matte hintereinander zu schalten. In jedem Fall sollten aber die Litzen aller Litzenanordnungen in exakt parallelen Strängen mit gleichen Abständen angeordnet sein. Dank der unterschiedlichen Frequenzbeaufschlagungen in den verschiedenen Bereichen kann den individuellen Bedürfnissen der Patienten besser Rechnung getragen werden und mit gleicher oder sogar kürzerer Behandlungsdauer ein besseres Therapieergebnis erzielt werden. Ganz wesentlich dafür trägt der sich immer wieder von oben nach unten fortschreitende Magnetfeldaufbau bei.

Zwar liegen auch bei dem eingangs beschriebenen Magnetfeldtherapiegerät die Litzenanordnungen etwa auf gleichem Niveau, jedoch sind sie in den Kanälen arretiert, was für die Haltbarkeit der Matte nachteilig ist, da die empfindlichen Litzen beim Zusammenlegen und Falten der Matte mechanisch stark beansprucht werden. Dank der Längsbeweglichkeit der parallelen Stränge in den Kanälen der Matte können sich die Stränge beim Falten der Matte in den Kanälen verschieben. Die Verbindungsbögen der parallelen Stränge liegen auf erhöhtem Niveau und stellen daher Entspannungszonen für die Litzenanordnung dar. Dank dieser Bauweise wird die Bruch- und Reißgefahr der Litzen wesentlich reduziert, wenn nicht gar eliminiert.

Anhand der Zeichnung, die ein Ausführungsbeispiel der Erfindung darstellt, wird diese näher beschrieben.

Es zeigt:

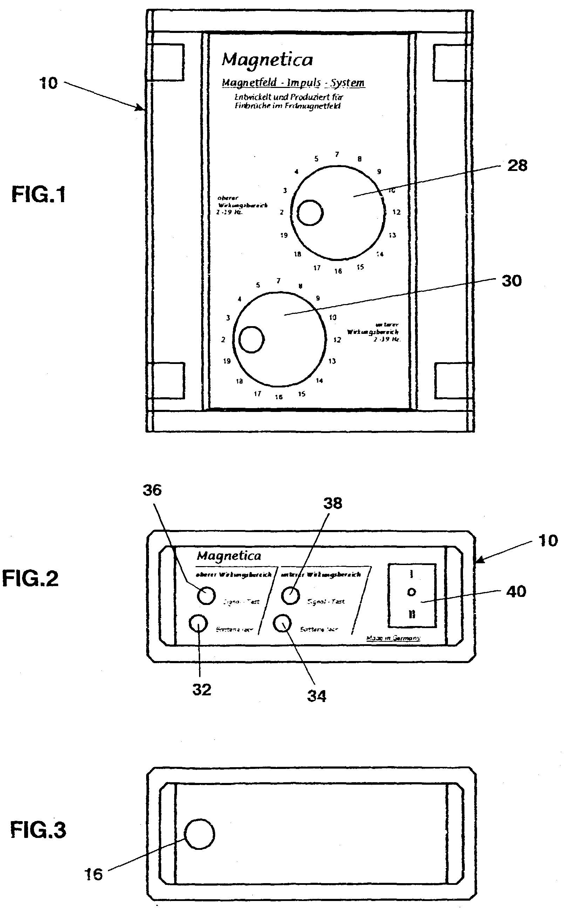

FIG. 1 eine Draufsicht auf das Steuergerät der Therapieeinrichtung,
FIG. 2 eine Vorderansicht des Steuergerätes
FIG. 3 eine rückseitige Ansicht des Steuergerätes,

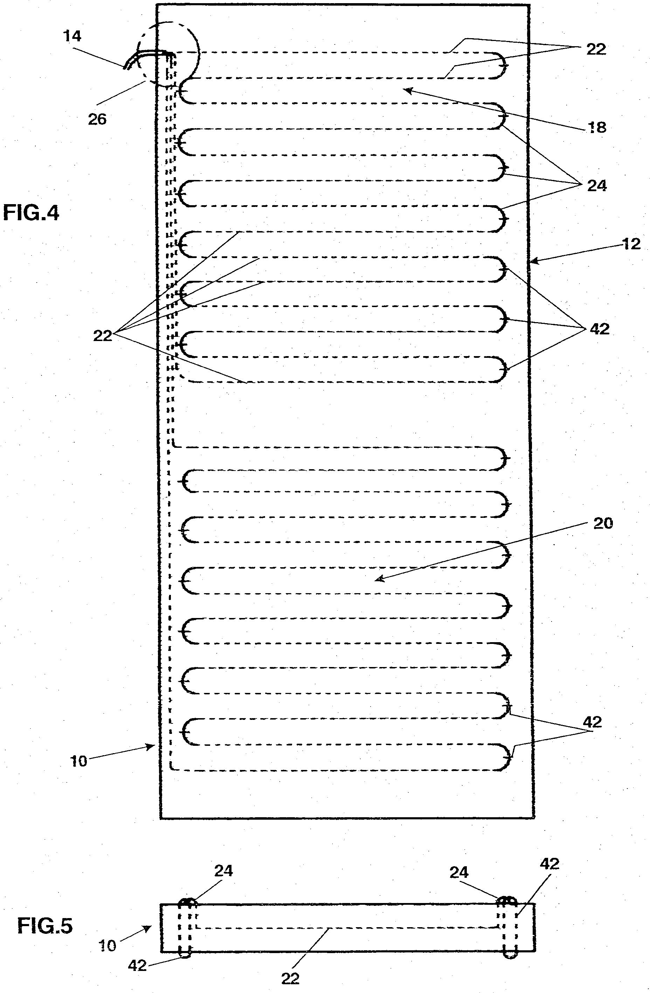

FIG. 4 eine Draufsicht auf eine Therapiematte und
FIG. 5 eine Stirnansicht der Therapiematte gemäß FIG. 4.

Die neue Therapieeinrichtung besteht aus einem Steuergerät 10 und einer Therapiematte 12, die über ein mehradriges Kabel 14 mit einem mehrpoligen Stecker und einer Steckbuchse 16 an der Rückseite des Steuergerätes 10 angeschlossen ist. Die Matte 12 ist länglich und die Länge beträgt etwa das Doppelte ihrer Breite. In der Matte 12 sind zwei Litzenanordnungen 18, 20 gleicher Größe eingearbeitet, die je aus einer Anzahl paralleler Stränge 22 und diese an ihren Enden abwechselnd miteinander verbindenden Verbindungsbögen 24 besteht, sodaß jede der beiden Litzenanordnungen 18, 20 schlangenförmig verläuft. Die parallelen Litzenstränge 22 verlaufen rechtwinklig zur Längserstreckung der Matte 12 und die Enden jeder Litzenanordnung 18 bzw. 20 sind zu einem gemeinsamen Mattenauslaß 26 geführt und setzen sich in dem Kabel 14 fort. Die Litzen weisen einen Isoliermantel auf.

Jeder Litzenanordnung 18 und 20 ist ein Frequenzeinstellknopf 28 bzw. 30 zugeordnet. Beide Knöpfe 28, 30 beeinflußen eine Steuerelektronik im Steuergerät 10, um die Litzenanordnungen 18, 20 mit Stromimpulsen im Bereich von 2 Hz und 19 Hz zu versorgen. Im Steuergerät sind nicht nur die Steuerkreise für die Impulserzeugung beider Litzenanordnungen 18, 20 getrennt, sondern für jede der beiden Litzenanordnungen ist auch eine eigene Stromversorgung z.B. in Form eines wiederaufladbaren Akkus im Steuergerät 10 untergebracht. Die beiden unteren Signaldioden 32, 34 an der Vorderseite des Steuergerätes 10 leuchten auf, wenn die jeweilige Batteriespannung ein Mindestmaß unterschritten hat. Die beiden jeweils darüber angeordneten Dioden 36, 38 blinken im Testbetrieb mit der an den Einstellknöpfen 28, 30 jeweils eingestellten Frequenz. Der Hauptschalter 40 am Steuergerät hat eine mittlere Nullstellung, eine Einschaltstellung und eine Teststellung. Die Signaldioden 36, 38 blinken nur in der Teststellung.

Wie sich aus FIG. 5 ergibt, liegen die parallelen Stränge 22 auf mittlerer Höhe der Matte 12. Während es auf eine genaue Einhaltung dieser Mittenlage nicht so sehr ankommt, ist wichtig, daß die Vielzahl paralleler Stränge 22 auf demselben Niveau liegt. Die Verbindungsbögen 24 befinden sich dagegen auf erhöhtem Niveau, nämlich auf der Deckseite der Matte 12. Im dargestellten Ausführungsbeispiel sind die parallelen Stränge 22 und die Verbindungsbögen 24 jeweils durch lotrechte Abknickungen verbunden. In der Praxis jedoch werden solche Abknickungen vermieden, vielmehr verlaufen die Verbindungen zwischen den Strängen 22 und den Bogen 24 schräg oder noch besser S-förmig. Die Scheitel der Verbindungsbögen 24 werden deckseitig an der Matte 12 mittels separater Verbindungselemente 42 gehaltert. Diese Verbindungselemente 42 wirken dabei lediglich als Niederhalter für die Verbindungsbögen 24, ohne diese festzuklammern.

Zur Montage der Litzenanordnungen 18, 20 werden in die Matte 12 in exakt gleichen Abständen von z.B. 3 cm Querschlitze eingebracht und zwar mit exakt gleicher Tiefe etwa gleich der halben Mattenstärke. Die Querschlitze weisen von beiden Längsrändern der Matte 12 einen Abstand auf. In diesen Querschlitzen werden nun die parallelen Stränge 22 der Litzenanordnung 18 bzw. 20 positioniert, sodaß sie an den Schlitzböden aufliegen. Im Bereich der Schlitzenden steigen die Litzen zu den Verbindungsbögen 24 hin an. Dann werden die einander benachbarten Schlitzbegrenzungsflächen oberhalb der eingelegten Litzenstränge 22 miteinander verklebt, wobei darauf zu achten ist, daß die Litzenstränge 22 in den so gebildeten Kanälen der Matte 12 längsbeweglich bleiben. Die Anordnung der Verbindungsbögen 24 auf höherem Niveau als die geradlinigen parallelen Stränge 22 mit der beschriebenen Halterung der Bögen auf der Deckseite der Matte 12 stellt für die Stränge 22 der Litzenanordnungen 18, 20 Entlastungszonen bereit, mit der Folge, daß die Matte eng zusammengerollt werden kann, ohne daß für die eingebauten Litzenanordnungen 18, 20 Bruchgefahr besteht.

Obwohl in der Zeichnung nur eine Ausführungsform einer Matte 12 mit zwei gleich großen Litzenanordnungen in beiden Längshälften der Matte 12 gezeigt ist, versteht sich, daß auch eine Drei- oder Mehrzahl-Bereichsausführung unter den Schutz der Erfindung fällt, bei der also im oberen Drittel, im mittleren Drittel und im unteren Drittel jeweils eine Litzenanordnung eingebaut ist. Dabei müssen die von den Litzenanordnungen beaufschlagten Bereiche nicht notwendigerweise gleich lang sein. Als vorteilhaft wird es dagegen angesehen, wenn zwischen je zwei Litzenanordnungen ein Abstand verbleibt, der größer als derjenige zwischen den parallelen Strängen 22 einer Litzenanordnung ist.

Im praktischen Einsatz werden zuerst die Frequenzeinstellknöpfe 28, 30 auf die gewünschten Arbeitsfrequenzen eingestellt. Dann wird der Schalter 40 auf Testbetrieb gestellt, sodaß an den Signaldioden 36, 38 die Blinkfrequenz überprüft werden kann, die mit der tatsächlichen Frequenz in den Litzenanordnungen 18, 20 übereinstimmt. Der Schalter 40 wird dann auf "Funktion" des Gerätes umgestellt.

Während bei dem beschriebenen Ausführungsbeispiel immer beide Behandlungsbereiche gleichzeitig aktiviert sind, liegt es im Rahmen der Erfindung, das Gerät 10 zusätzlich mit einem Wahlschalter oder mit Wahlschaltern für jede der Litzenanordnungen 18, 20 auszustatten, sodaß nur der obere Bereich, nur der untere Bereich oder auch beide Bereiche gemeinsam aktiviert werden können. Schließlich liegt es auch im Rahmen der Erfindung, das Steuergerät 10 mit mindestens einem Zeitschalter auszustatten, vorzugsweise jedoch mit einem Zeitschalter für jeden Behandlungsbereich. Damit ist es möglich, die Behandlungsdauer für die einzelnen Bereiche unterschiedlich vorzugeben.

## Patentansprüche

1. Magnetfeldtherapiegerät mit einer länglichen Matte (12), in der mehrere flexible, gleich breite, elektrisch leitende Litzenanordnungen (18; 20) je mit einer Vielzahl paralleler Stränge (22) in Kanälen der Matte schlangenförmig verlegt und die parallelen Stränge (22) der Litzenanordnungen (18, 20) rechtwinklig zur Längserstreckung der Matte (12) orientiert und in Längsrichtung der Matte (12) hintereinander angeordnet sind, deren Enden über Kabel (14) an einen Stromimpulserzeuger angeschlossen oder anschließbar sind, **dadurch gekennzeichnet, daß** die parallelen Stränge (22) der Litzenanordnungen (18, 20) rechtwinklig zur Längserstreckung der Matte (12) orientiert und in allen Litzenanordnungen (18, 20) in exakt gleichem Abstand im Bereich von 2 bis 5 cm, insbesondere etwa 3 cm zueinander angeordnet und in den Kanälen längsbeweglich geführt sind, und daß die Verbindungsbögen (24) der Litzenanordnungen (18, 20) auf einem vom Niveau der parallelen Stränge (22) beabstandeten Niveau liegen.

2. Gerät nach Anspruch 1, **dadurch gekennzeichnet, daß** die Verbindungsbögen (24) auf der Oberfläche der Matte (12) liegen und mittels jeweils eines, die Matte durchsetzenden Verbindungselementes (42) an dies er gehaltert sind.

3. Gerät nach Anspruch 2, **dadurch gekennzeichnet, daß** die Verbindungsbögen (24) an den Verbindungselementen (42) ein Bewegungsspiel aufweisen.

4. Gerät nach einem der Ansprüche 1 bis 3, **dadurch gekennzeichnet, daß** die Matte (12) mit einer Anzahl Querschlitze gleich der Anzahl der parallelen Stränge (22) versehen ist, daß die Tiefe der Querschlitze einen Wert im Bereich von 30 % bis 60 % der Mattendicke hat, daß die Querschlitze im Abstand von beiden Mattenlängsrändern enden, daß die parallelen Stränge (22) der Litzenanordnungen (18, 20) im Bodenbereich der Querschlitze liegen und daß die Schlitzbegrenzungsflächen der Matte oberhalb der parallelen Stränge (22) paarweise miteinander verbunden, insbesondere verklebt sind.

5. Gerät nach einem der Ansprüche 1 bis 4, **dadurch gekennzeichnet, daß** die beiden benachbarten parallelen Endstränge (22) zweier Litzenanordnungen (18, 20) einen größeren Abstand aufweisen als die Stränge (22) jeder Litzenanordnung (18; 20).

6. Gerät nach einem der Ansprüche 1 bis 5, **dadurch gekennzeichnet, daß** die Matte (12) zwei gleich große Litzenanordnungen (18, 20) aufweist, die je in einer Längshälfte der Matte (12) eingebaut sind.

7. Gerät nach einem der Ansprüche 1 bis 6, **dadurch gekennzeichnet, daß** jede Litzenanordnung (18, 20) an einen eigenen Stromimpulserzeuger angeschlossen oder anschließbar ist.

8. Gerät nach einem der Ansprüche 1 bis 7, **dadurch gekennzeichnet, daß** für jeden Impulserzeuger eine eigene Stromquelle, insbesondere Batterie oder Akku vorgesehen ist.

9. Gerät nach einem der Ansprüche 1 bis 8, **dadurch gekennzeichnet, daß** ein über ein mehradriges Kabel (14) mit der Matte (12) verbundenes Steuergerät (10) neben der Stromversorgung, den Impulserzeugern, Reglern und der Steuerelektronik für jede eingebaute Stromquelle einen eigenen Spannungsüberwachungskreis mit Anzeigedioden (32, 34) bei Unterschreitung einer Mindestspannung aufweist.
